# EUROPEAN PATENT APPLICATION

(11) **EP 4 538 283 A1**
(43) Date of publication of application: **16.04.2025**
(21) Application number: 23827531.7
(22) Date of filing: 22.06.2023
(51) Int. Cl.: C07K 14/195, C12N 15/77, C12P 13/24

(54) **L-HISTIDINE EXPORT PROTEIN AND METHOD FOR PRODUCING L-HISTIDINE BY USING SAME**

(30) Priority: 23.06.2022 KR 20220076773
(71) Applicant: CJ CheilJedang Corporation, Seoul 04560 (KR)
(72) Inventor: HUH, Lan, Seoul 04560 (KR); SEO, Chang Il, Seoul 04560 (KR); CHEONG, Ki Yong, Seoul 04560 (KR)
(74) Representative: Meissner Bolte Partnerschaft mbB
(86) International application number: PCT/KR2023/008656
(87) International publication number: WO 2023/249421

(57) **Abstract**

The present application discloses a histidine export protein or a variant thereof having the ability to export L-histidine, which can dramatically enhance the production of L-histidine when expressed in a microorganism having the ability to produce L-histidine.

## Description

### [TECHNICAL FIELD]

### Cross-reference to related application(s)

The present application claims the benefit of priority based on Korean patent application No. 10-2022-0076773 filed on June 23, 2022, and the entire contents described in the documents of the corresponding Korean patent application are incorporated as part of this specification.

The present application relates to a novel protein having histidine export activity, an L-histidine producing microorganism modified to express the protein, and a method for producing L-histidine using the microorganism.

### [BACKGROUND ART]

L-histidine is one amino acid of 20 standard amino acids, and from a nutritional point of view, it is not required in a large amount for adults, but it is classified as an essential amino acid for growing children. In addition, L-histidine is involved in important physiological processes such as antioxidation and immunomodulation and the like, and is used in the medical industry such as raw materials of gastrointestinal therapeutics and circulatory therapeutics and amino acid infusion preparations, and the like.

L-histidine is mainly produced through protein hydrolysis extraction using blood meal as a raw material, as it is largely included in hemoglobin. However, this method has disadvantages such as low efficiency and environmental pollution and the like. On the other hand, it is possible to produce L-histidine through microbial fermentation, but large-scale industrialization has not been achieved. This is because the biosynthesis of L-histidine is competitive with phosphoribosyl pyrophosphate (PRPP), which is a nucleotide synthesis precursor, and has a biosynthesis process and a regulatory mechanism that are complex and require high energy.

An example that production of the corresponding amino acids is increased, when expression and/or functions of proteins having export ability of different kinds of amino acids are enhanced, has been known, but there has been little prior research on proteins having L-histidine-specific export ability.

Under this background, the discovery of proteins having histidine-specific export ability and development of technology for producing histidine using them are required.

### [DISCLOSURE]

### [TECHNICAL PROBLEM]

An object of the present application is to provide a mutant L-histidine export protein, in which (1) the amino acid corresponding to the 72th amino acid residue of the amino acid sequence of SEQ ID NO: 43 is substituted with another amino acid, or
(2) the amino acid corresponding to the 124th residue of the amino acid sequence of SEQ ID NO: 43 is substituted with another amino acid, or
(3) both of the (1) and (2) are substituted.

Another object of the present application is to provide a microorganism comprising the protein or a polynucleotide encoding the protein.

Other object of the present application is to provide a composition for producing L-histidine comprising the protein, the polynucleotide encoding the protein, or the microorganism.

Other object of the present application is to provide a use of the protein, the polynucleotide encoding the protein, or the microorganism, for using in production of L-histidine.

Other object of the present application is to provide a use of the protein, the polynucleotide encoding the protein, or the microorganism, for using in preparation of a composition for producing L-histidine.

Other object of the present application is to provide a method for producing L-histidine, comprising culturing the microorganism in a medium.

### [TECHNICAL SOLUTION]

The present application suggests that the production of L-histidine can be dramatically enhanced, as a result of discovering a variant of a histidine export protein having L-histidine export ability, and expressing this in a microorganism having production ability of L-histidine.

In the present description, it has been confirmed that a microorganism expressing AzIC family ABC transporter permease derived from *Helcobacillus massiliensis* has excellent production ability of L-histidine, and it has been confirmed that the production ability of L-histidine is further increased, when an amino acid substitution mutation is introduced into a specific position of the AzIC family ABC transporter permease.

### Protein, polynucleotide, and recombinant vector

One aspect provides a mutant protein (or polypeptide) having L-histidine export activity. The protein may be a protein having L-histidine-specific export ability. In the present description, the mutant protein may be represented by a mutant histidine export protein. The mutant protein may have AzIC family ABC transporter permeation activity.

In one embodiment, the mutant protein may have L-histidine export activity equivalent to or enhanced more than wild-type L-histidine export protein (for example, AzID domain-containing protein, or AlzC family ABC transporter permease, etc.). The AzID domain-containing protein or AzIC family ABC transporter permease protein may be derived from *Helcobacillus massiliensis.* In the present description, the AzID domain-containing protein derived from *Helcobacillus massiliensis* may be described as HmaE protein (or HmaE), and the AzIC family ABC transporter permease protein derived from *Helcobacillus massiliensis* may be described as HmaF protein (or HmaF).

In one embodiment, the mutant protein (for example, mutant protein of the AzID domain-containing protein, specifically, mutant protein of the AzID domain-containing protein derived from *Helcobacillus massiliensis*) may be expressed together with the AzIC family ABC transporter permease protein in the same operon gene. In one embodiment, the mutant protein may have L-histidine export activity by binding to the AzIC family ABC transporter protein.

In one embodiment, the mutant protein may be a mutant protein of the AzIC family ABC transporter permease protein derived from *Helcobacillus massiliensis.*

The mutant protein may be a mutant protein in which a mutation that one or more amino acid residues of wild-type AzIC family ABC transporter permease protein derived from *Helcobacillus massiliensis* are substituted, deleted, or inserted.

The wild-type AzIC family ABC transporter permease protein derived from *Helcobacillus massiliensis* may comprise the amino acid sequence of SEQ ID NO: 43 (WP_055090792.1), or consist of the sequence.

In one embodiment, the mutant protein may be one in which (1) the amino acid corresponding to the 72th residue is substituted with another amino acid, or
(2) the amino acid corresponding to the 124th residue is substituted with another amino acid, or
(3) both of the (1) and (2) are substituted, from the N-terminus in the amino acid sequence of SEQ ID NO: 43.

As above, counting amino acids from the N-terminus in the amino acid sequence, may mean counting with methionine (Met, M) translated from the start codon as the first amino acid.

In one embodiment, the mutant protein (1) may comprise a sequence in which the amino acid corresponding to the 72th residue is substituted with another amino acid, which is Leucine (Leu, L), arginine (Arg, R), histidine (His, H), lysine (Lys, K), aspartic acid (Asp, D), glutamic acid (Glu, E), serine (Ser, S), threonine (Thr, T), asparagine (Asn, N), glutamine (Gln, Q), cysteine (Cys, C), glycine (Gly, G), proline (Pro, P), alanine (Ala, A), valine (Val, V), methionine (Met, M), phenylalanine (Phe, F), tyrosine (Tyr, Y), or tryptophan (Trp, W), that is, an amino acid different from the original amino acid, or
(2) comprise a sequence in which the amino acid corresponding to the 124th residue is substituted with another amino acid, which is valine, arginine, histidine, lysine, aspartic acid, glutamic acid, serine, threonine, asparagine, glutamine, cysteine, glycine, proline, alanine, leucine, methionine, phenylalanine, tyrosine, or tryptophan, that is, an amino acid different from the original amino acid, or,
(3) comprise a sequence in which both of the (1) and (2) are substituted, from the N-terminus in the amino acid sequence of SEQ ID NO: 43.

In one embodiment, the mutant protein may comprise a sequence in which (1) the amino acid corresponding to the 72th residue is substituted with leucine, glycine, proline, alanine, valine, or methionine;
(2) the amino acid corresponding to the 124th residue is substituted with valine, glycine, proline, alanine, leucine, or methionine; or
(3) both of the (1) and (2) are substituted, from the N-terminus in the amino acid sequence of SEQ ID NO: 43.

In one specific embodiment, the mutant protein may be one in which the amino acid corresponding to the 72th residue is substituted with leucine, and the 124th residue is substituted with valine, from the N-terminus in the amino acid sequence of SEQ ID NO: 43. It is obvious that even if some amino acid sequences other than the amino acids corresponding to the 72th and/or 124th amino acid residues are deleted, modified, substituted, or added, from the N-terminus of SEQ ID NO: 43 in the mutant protein, they can be included in the mutant protein of the present application, as long as they exhibit AzIC family ABC transporter permease activity.

In addition, in one embodiment, the mutant protein may comprise a polypeptide in which (1) the amino acid corresponding to the 72th residue is substituted with another amino acid, or (2) the amino acid corresponding to the 124th residue is substituted with another amino acid, or (3) both of the (1) and (2) are substituted, from the N-terminus in the amino acid sequence of SEQ ID NO: 43, in the amino acid sequence having sequence homology or sequence identity of at least 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, 99% or more, or 99.5% or more to the amino acid sequence described as SEQ ID NO: 43. In other words, a polypeptide which comprises a substitution at the positions corresponding to the 72th and/or 124th residues from the N-terminus in the amino acid sequence of SEQ ID NO: 43 with another amino acid, and has sequence homology or sequence identity of at least 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, 99% or more, or 99.5% or more to the amino acid sequence of SEQ ID NO: 43, and has AzIC family ABC transporter permease activity, may be included in the mutant protein of the present application.

In one specific embodiment, the mutant protein may comprise the amino acid sequence of SEQ ID NO: 56 or consist of the sequence, but not limited thereto. It is obvious that even if some amino acid sequences other than the 72th and/or 124th amino acid residues are deleted, modified, substituted, or added, from the N-terminus of SEQ ID NO: 56 in the mutant protein, they can be included in the mutant protein of the present application, as long as they exhibit AzIC family ABC transporter permease activity. In addition, in one embodiment, the mutant protein may comprise a polypeptide which the amino acids corresponding to the 72th and/or 124th residues from the N-terminus in the amino acid sequence of SEQ ID NO: 56 are fixed in, and has sequence homology or sequence identity of at least 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, 99% or more, or 99.5% or more to the amino acid sequence of SEQ ID NO: 56. In other words, a polypeptide which has a substitution of the amino acids corresponding to the 72th and/or 124th residues from the N-terminus in the amino acid sequence of SEQ ID NO: 56 into other amino acids, and has homology or identity of at least 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, 99% or more, or 99.5% or more to the amino acid sequence of SEQ ID NO: 56, and has AzIC family ABC transporter permease activity, may be included in the mutant protein of the present application.

In one embodiment, the mutant protein may have enhanced L-histidine export activity more than a wild-type protein (for example, wild-type AzID domain-containing protein). In one embodiment, the mutant protein may have further enhanced L-histidine export activity, when expressed with a wild-type AzIC family ABC transporter permease protein.

The wild-type L-histidine export protein may be a protein having sequence homology of 60% or more to SEQ ID NO: 43 (wild-type AzIC family ABC transporter permease protein derived from *Helcobacillus massiliensis*), SEQ ID NO: 44 (wild-type AzID domain-containing protein derived from *Helcobacillus massiliensis*) or a combination thereof. For example, in one specific embodiment, the wild-type L-histidine export protein may have homology of 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, 99% or more, or 99.5% or more to SEQ ID NO: 43, 44, or a combination thereof. The protein represented by SEQ ID NO: 43 may be encoded by the nucleic acid sequence of SEQ ID NO: 45, and the protein represented by SEQ ID NO: 44 may be encoded by the nucleic acid sequence of SEQ ID NO: 46, or the proteins represented by SEQ ID NO: 43 and/or SEQ ID NO: 44 may be encoded by the nucleic acid sequence of SEQ ID NO: 47 (an operon sequence fused at the overlapping region of the 3' end of SEQ ID NO: 45 and the 5' end of SEQ ID NO: 46).

Another aspect, provides a polynucleotide encoding (coding) the mutant protein.

In the present application, the term, "polynucleotide" means a polymer of nucleotides in which nucleotide monomers are connected long in a chain shape by covalent bonds, a DNA or RNA strand of a certain length or longer, and more specifically, a polynucleotide fragment encoding the mutant polypeptide.

The polynucleotide encoding the mutant protein of the present application may comprise a base sequence encoding the amino acid sequence of SEQ ID NO: 56.

In the present description, that a polynucleotide (may be interchangeably used with "gene") or polypeptide (may be interchangeably used with "protein") "comprises a specific nucleic acid sequence or amino acid sequence, consists of a specific nucleic acid sequence or amino acid sequence, or is represented by a specific nucleic acid sequence or amino acid sequence" may mean that the polynucleotide or polypeptide essentially comprises the specific nucleic acid sequence or amino acid sequence, and may be interpreted as comprising "a substantially equivalent sequence" in which a mutation (deletion, substitution, modification, and/or addition) is applied to the specific nucleic acid sequence or amino acid sequence (or not excluding the mutation), within a range of maintaining the original functions and/or desired functions of the polynucleotide or polypeptide.

In one embodiment, the nucleic acid sequence or amino acid sequence provided in the present description may comprise one modified by common mutagenesis, for example, direct evolution and/or site-directed mutagenesis and the like, within a range of maintaining the original functions and/or desired functions thereof. In one embodiment, that a polynucleotide or polypeptide "comprises a specific nucleic acid sequence or amino acid sequence or consists of a specific nucleic acid sequence or amino acid sequence" may mean that the polynucleotide or polypeptide (i) essentially comprises the specific nucleic acid sequence or amino acid sequence, or (ii) consists of an amino acid sequence having homology of 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, 99% or more, or 99.5% or more to the specific nucleic acid sequence or amino acid sequence or essentially comprises thereof, and maintain the original functions and/or desired functions. In the present description, the desired functions may mean giving or increasing L-histidine export activity and/or L-histidine production ability of a microorganism.

In the nucleic acid sequence described in the present description, various modification may be made in the coding region within a range that does not change the amino acid sequence and/or function of the protein expressed from the coding region, in consideration of a codon preferred in a microorganism in which the protein is to be expressed, due to codon degeneracy.

In the present description, the term "homology (identity)" may refer to the degree of being consistent to the given nucleic acid sequence or amino acid sequence, and may be expressed as a percentage (%). In case of identity to a nucleic acid sequence, for example, it may be determined using algorithm BLAST (See: Karlin and Altschul, Pro. Natl. Acad. Sci. USA, 90, 5873, 1993) or FASTA by Pearson (See: Methods Enzymol., 183, 63, 1990). Based on this algorithm BLAST, programs called BLASTN or BLASTX have been developed (See: http://www.ncbi.nlm.nih.gov).

In one embodiment, the polynucleotide comprising the specific nucleic acid sequence provided in the present description may be interpreted as including not only the specific nucleic acid sequence or a nucleic acid sequence substantially equivalent thereto, but also a polynucleotide fragment comprising a nucleic acid sequence complementary to the specific nucleic acid sequence. Specifically, the complementary polynucleotide may be hybridized at an Tm value which can be adjusted by those skilled in the art depending on the purpose, for example, a Tm value of 55°C, 60°C, 63°C or 65°C, and be analyzed under conditions described below: These conditions are specifically described in known documents. For example, a condition of hybridizing genes having high complementarity of 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, 99% or more, or 99.5% or more, and not hybridizing genes having complementarity lower than that, or a washing condition of common southern hybridization, which is a condition of washing at the salt concentration and temperature corresponding to 60°C, 1x SSC(saline-sodium citrate buffer), and 0.1%(w/v) SDS (Sodium Dodecyl Sulfate); 60°C, 0.1x SSC, and 0.1% SDS; or 68°C, 0.1x SSC, and 0.1% SDS, once, specifically, twice to three times may be listed, but not limited thereto. Hybridization requires that two nucleotides have complementary sequences, or a mismatch between bases may be allowed depending on the stringency of hybridization. The term "complementary" may be used to describe the relationship between nucleotide bases that can be hybridized with each other. For example, in case of NDA, adenine is complementary to thymine, and cytosine is complementary to guanine. The appropriate stringency to hybridize polynucleotides depends on the length and the degree of complementarity of the polynucleotides, and this is well known in the art (See Sambrook et al., supra,9.50-9.51, 11.7-11.8).

Introduction of the polynucleotide or vector may be performed by selecting a known transformation method appropriately by those skilled in the art. In the present description, the term "transformation" is a process of introducing a specific polynucleotide or a vector comprising the same into a host cell, and the transformed polynucleotide may be positioned as inserted into chromosome or be positioned outside the chromosome in the host cell. As one example, transformation may introduce a polynucleotide encoding a target protein (foreign protein) or a vector comprising the same into a host cell so that the protein encoded by the polynucleotide is expressed in the host cell. In addition, the polynucleotide may comprise DNA and/or RNA encoding a target protein. As long as the polynucleotide can be introduced into a hots cell and expressed, there is no limitation on the form in which it is introduced. For example, the polynucleotide may be introduced into a host cell in a form of an expression cassette, which is a genetic construct comprising all elements required to be expressed by itself. The expression cassette may commonly comprise expression regulatory elements such as a promoter operably linked to the polynucleotide, a transcription termination signal, a ribosome binding site and/or a translation termination signal and the like. The expression cassette may be in a form of an expression vector capable of self-replication. In addition, the polynucleotide may be introduced into a host cell in its own form and be operably linked to a sequence necessary for expression in the host cell.

In the above, the term "operably linked" may mean that expression regulatory elements (e.g., promoter) and a polynucleotide are functionally linked so that the expression regulatory elements perform transcription regulation (e.g., transcription initiation) of a polynucleotide encoding a target protein (foreign protein). Operable linking may be performed using genetic recombination technology known in the art, and for example, it may be performed by common site-specific DNA cleavage and ligation, but not limited thereto.

A method for transforming the polynucleotide into a host cell may be performed by any method for introducing a nucleic acid into a cell (microorganism), and depending on the host cell, it may be performed by appropriately selecting transformation technology known in the art. As the known transformation method, electroporation, calcium phosphate (CaPO4) precipitation, calcium chloride (CaCl2) precipitation, microinjection, polyethylene glycol (PEG) precipitation (polyethylene glycol-mediated uptake), DEAE-dextran method, cation liposome method, lipofection, lithium citrate-DMSO method, and the like may be exemplified, but not limited thereto.

Introduction (insertion) of the polynucleotide into host cell genome (chromosome) may be performed by selecting a known method appropriately by those skilled in the art, and for example, it may be performed using an RNA-guided endonuclease system (or CRISPR system; for example, at least one selected from the group consisting of mixtures (for example, mixture of the RNA-guided endonuclease protein and guide RNA, etc.), complexes (for example, ribonucleic acid fusion proteins (RNP), recombinant vectors (for example, vector comprising an RNA-guided endonuclease encoding gene and guide RNA encoding DNA together, etc.), and the like, which comprise (a) RNA-guided endonuclease (e.g., Cas9 protein, etc.), a gene encoding thereof, or a vector comprising the gene; and (b) a guide RNA (e.g., single guide RNA (sgRNA), etc.), DNA encoding thereof, or a vector comprising the DNA), but not limited thereto.

Other aspect, provides a recombinant vector comprising the polynucleotide. The recombinant vector may be used as an expression vector of the polypeptide. The recombinant vector may be for inserting the polynucleotide into genome of a host cell or substituting the corresponding gene of the host cell genome.

In the present description, the term "vector" means a DNA product containing the base sequence of a polynucleotide encoding the target protein operably linked to a suitable regulatory sequence so as to express a target protein in a suitable host. The regulatory sequence may comprise a promoter that can initiate transcription, any operator sequence for regulating transcription, a sequence encoding a suitable mRNA ribosome binding site, and/or a sequence that regulates termination of transcription and/or translation. The vector may be expressed independently of the genome of the host cell, or be integrated into genome of the host cell, after being transformed into a suitable host cell.

The vector that can be used in the present description is not particularly limited as long as it is replicable in a host cell, and it may be selected from all vectors commonly used. Examples of the commonly used vectors may include plasmids, cosmids, viruses, bacteriophages, and the like, in a natural state or a recombinant state. For example, as the vector, pWE15, M13, MBL3, MBL4, IXII, ASHII, APII, t10, t11, Charon4A, and Charon21A and the like may be used as the phage vector or cosmid vector, and pBR-series, pUC-series, pBluescriptll-series, pGEM-series, pTZ-series, pCL-series and pET-series and the like may be used as the plasmid vector. Specifically, pDZ, pACYC177, pACYC184, pCL, pECCG117, pUC19, pBR322, pMW118, pCC1BAC vectors and the like may be exemplified, but not limited thereto.

The vector that can be used in the present description may be a known expression vector and/or a vector for insertion into host cell chromosome of a polynucleotide. Insertion of the polynucleotide into host cell chromosome may be conducted by any method known in the art, for example, homologous recombination or CRISPR system, but not limited thereto. The vector may further comprise a selection marker for confirming whether it is inserted into the chromosome. The selection marker is to select a cell transformed with the vector, that is, to confirm insertion of the polynucleotide, and it may be selected from genes giving selectable phenotypes such as drug resistance, auxotrophy, resistance to cytotoxic agents or expression of surface proteins and used. Since only the cells expressing the selection marker are survived or show different phenotypes, the transformed cells may be selected.

### Microorganism

Other aspect, provides a microorganism comprising at least one (one kind, 2 kinds, or all of the 3 kinds) selected from the group consisting of the mutant protein, a polynucleotide encoding the mutant protein, and a recombinant vector comprising the polynucleotide. The microorganism may have L-histidine export activity and/or L-histidine production ability. The microorganism may have enhanced (or increased, improved) L-histidine export activity and/or L-histidine production ability compared with a microorganism not comprising at least one selected from the group consisting of the mutant protein, a polynucleotide encoding the mutant protein, and a recombinant vector comprising the polynucleotide.

The mutant protein may be foreign. In the present description, "foreign" may mean not one which is present endogenously in the microorganism, but derived from a species other than the microorganism.

In the present description, "microorganism with enhanced L-histidine export activity and/or L-histidine production ability" may be that a microorganism having no L-histidine export activity and/or L-histidine production ability has L-histidine export activity and/or L-histidine production ability, or has L-histidine export activity and/or L-histidine production ability higher than the original L-histidine export activity and/or L-histidine production ability, by being engineered (mutated) to express the afore-mentioned mutant protein.

In the present description, "microorganism" encompasses unicellular bacteria, and may be interchangeably used with "cell".

In the present application, the term, "microorganism (or, strain)" includes all of wild-type microorganisms or microorganisms in which genetic modification has occurred naturally or artificially, and may be a microorganism in which a specific mechanism is weakened or enhanced, due to causes that a foreign gene is inserted or activity of an endogenous gene is enhanced or inactivated, and the like, which is a microorganism comprising a genetic modification for production of a desired polypeptide, protein, or product (for example, L-histidine).

The microorganism of the present application mya be a genetically modified microorganism (for example, recombinant microorganism) to enhance activity of L-histidine export protein or a polynucleotide encoding the same, but not limited thereto. The vector is as described above.

In the present description, the microorganism before mutated to express the mutant protein may be expressed as "parent strain (parent microorganism) or host cell", to distinguish from the mutated microorganism.

That the microorganism (or strain, recombinant cell) has L-histidine export activity and/or L-histidine production ability, or has enhanced L-histidine export activity and/or L-histidine production ability, may mean that the L-histidine export activity and/or L-histidine production ability are given differently from a non-modified microorganism, a cell before recombination, a parent strain, and/or a wild-type strain, which have no L-histidine export activity and/or L-histidine production ability, or that the L-histidine export activity and/or L-histidine production ability are enhanced, compared with the non-modified microorganism, cell before recombination, parent strain, and/or wild-type strain.

The microorganism of the present application may be a microorganism comprising any one or more of the mutant protein of the present application, a polynucleotide encoding the mutant protein of the present application and a vector comprising the polynucleotide of the present application; a microorganism modified to express the mutant protein of the present application or the polynucleotide of the present application; a microorganism (for example, recombinant strain) expressing the mutant protein of the present application, or the polynucleotide of the present application; or a microorganism (for example, recombinant strain) having the activity of the mutant protein of the present application, but not limited thereto.

In one embodiment, the microorganism may be at least one selected from the group consisting of microorganisms of the genus *Corynebacterium,* microorganisms of the genus *Escherichia,* and the like. The microorganism of the genus *Corynebacterium* may include *Corynebacterium glutamicum, Corynebacterium ammoniagenes, Brevibacterium lactofermentum, Brevibacterium flavum, Corynebacterium thermoaminogenes, Corynebacterium efficiens,* and the like, but it is not necessarily limited thereto. Further more specifically, the microorganism of the genus *Corynebacterium* may be *Corynebacterium glutamicum.* The strain of the genus *Escherichia* may be *Escherichia coli.*

The microorganism may comprise at least one (1 kind, 2 kinds, or all of 3 kinds) selected from the group consisting of a mutant protein, a polynucleotide encoding the mutant protein, and a recombinant vector comprising the polynucleotide. In one embodiment, the mutation to express the mutant protein may be performed by introducing a polynucleotide encoding the afore-mentioned mutant protein, or a recombinant vector comprising the same, or be performed by artificial mutation (for example, Error-prone PCR, etc.), or the like. The polynucleotide encoding the mutant protein to be introduced into a parent strain as such may substitute a gene encoding AzIC family ABC transporter permease in the present strain or be further comprised in addition thereto.

In one embodiment, the microorganism may have further enhanced L-histidine export activity and/or L-histidine production ability, when it comprises the mutant protein with the wild-type AzIC family ABC transporter permease protein.

In one embodiment, the microorganism with enhanced L-histidine export activity and/or L-histidine production ability may have increased L-histidine production ability by about 10% or more, 15% or more, 20% or more, 30% or more, 40% or more, 50% or more, 60% or more, 70% or more, or 80% or more, compared with a parent strain before mutated, a non-modified microorganism, and a microorganism comprising a wild-type L-histidine export protein, but not limited thereto.

In other embodiment, the microorganism with enhanced L-histidine export activity and/or L-histidine production ability may have increased L-histidine production ability by about 1 g/L or more, 1.5 g/L or more, 2 g/L or more, 2.5 g/L or more, 3 g/L or more, 3.5 g/L or more, or 4 g/L or more, compared with a parent strain before mutated, a non-modified microorganism, and a microorganism comprising a wild-type L-histidine export protein, but not limited thereto.

The term "about" is a range that includes all such as ±0.5, ±0.4, ±0.3, ±0.2, ±0.1, and the like, and includes all numerical values equivalent or similar to the numerical value following the term about, but is not limited thereto.

Other aspect, provides a composition for producing L-histidine comprising the mutant protein, the polynucleotide, the recombinant vector or the microorganism.

Other aspect, provides a use of the mutant protein, the polynucleotide, the recombinant vector, or the microorganism, for using in production of L-histidine.

Other aspect provides a use of the mutant protein, the polynucleotide, the recombinant vector, or the microorganism, for using in preparation of a composition for producing L-histidine.

Other aspect, provides a method for producing (preparing) L-histidine, comprising culturing the microorganism in a medium. The method for preparing may further comprise recovering L-histidine from the cultured microorganism, medium, or both of them, after the culturing.

Other aspect, provides a method for increasing L-histidine export activity and/or L-histidine production ability of the microorganism, or a method for giving L-histidine export activity and/or LL-histidine production ability to the microorganism, comprising enhancing L-histidine export activity and/or L-histidine production ability of a microorganism.

The introducing a mutation my comprise introducing (transforming) a polynucleotide encoding the mutant protein or a recombinant vector comprising the polynucleotide into a microorganism, or comprise artificially generating a mutation (for example, Error-prone PCR, etc.).

Other embodiment provides a method for producing L-histidine, comprising culturing a microorganism with enhanced L-histidine export activity and/or L-histidine production ability in a medium. The method may further comprise recovering L-histidine from the cultured microorganism, medium, or both of them, after the culturing.

In the method, the culturing a microorganism is not particularly limited thereto, but it may be performed by a known batch culture method, continuous culture method, fed-batch culture method, or the like. At this time, the culture conditions, are not particularly limited thereto, but appropriate pH (for example, pH 5 to 9, specifically, pH 6 to 8, most specifically, pH 7.2) may be adjusted using a basic compound (e.g.: sodium hydroxide, potassium hydroxide, or ammonia) or acidic compound (e.g.: phosphoric acid, or sulfuric acid), and the aerobic condition may be maintained by introducing oxygen or an oxygen-containing gas mixture into the culture. The culture temperature may be maintained at 20 to 45°C, or 25 to 40°C, and it may be cultured for about 10 to 160 hours, but not limited thereto. L-histidine produced by the culturing may be secreted into the medium or remain within the cells.

The medium available for the culturing may use at least one individually or at least two in combination, which are selected from the group consisting of sugar and carbohydrates (e.g.: glucose, sucrose, lactose, fructose, maltose, molasse, starch and cellulose), oils and fats (e.g.: soybean oil, sunflower seed oil, peanut oil and coconut oil), fatty acids (e.g.: palmitic acid, stearic acid and linoleic acid), alcohols (e.g.: glycerol and ethanol), organic acids (e.g.: acetic acid) and the like, as a carbon source, but not limited thereto. As a nitrogen source, at least one individually or at least two in combination, which are selected from the group consisting of nitrogen-containing organic compounds (e.g.: peptone, yeast extract, broth, malt extract, corn steep liquor, soybean meal and urea), inorganic compounds (e.g.: ammonium sulfate, ammonium chloride, ammonium phosphate, ammonium carbonate, and ammonium nitrate) and the like, may be used, but it is not limited thereto. As a phosphorus source, at least one individually or at least two in combination, which are selected from the group consisting of potassium phosphate, potassium dihydrogen phosphate, dipotassium hydrogen phosphate, nitrogen-containing salts corresponding thereto and the like, may be used, but it is not limited thereto. In addition, the medium may comprise essential growth-promoting substances such as other metal salts (e.g.: magnesium sulfate, iron sulfate, manganese sulfate, etc.), amino acids, and/or vitamins and the like.

The recovering L-histidine may be collecting desired amino acids from a medium, cultured solution, or microorganism, using a suitable method known in the art depending on the culturing method. For example, the recovering may be performed by one or more methods selected from centrifugation, filtration, anion exchange chromatography, crystallization, HPLC, and the like. The method for recovering L-histidine may additionally comprise purifying before it, simultaneously, or after it.

### [ADVANTAGEOUS EFFECTS]

The present application discloses a histidine export protein or a variant thereof having the ability to export L-histidine, which can dramatically enhance the production of L-histidine when expressed in a microorganism having the ability to produce L-histidine.

### [MODE FOR INVENTION]

Hereinafter, the present invention will be described in more detail by the following examples. However, they are intended to illustrate the present invention only, but the scope of the present invention is not limited by these examples.

### Example 1. Foreign histidine export gene search and candidate selection

In order to select protein candidates having L-histidine-specific export ability, as a result of PSI-BLAST search based on NCBI and Kegg database, using amino acid sequences of amino acids by category (export proteins for basic amino acid: L-lysine (L-lys), aromatic amino acid: tryptophan (Trp), branched-chain amino acid: isoleucine (Ile) (LysE (Arch Microbiol 180: 155-160), Wex (Korean Registered Patent No. 10-1968317), BrnFE (Arch Microbiol 180: 155-160)) as query sequences, candidate genes predicted as membrane proteins that have possibility to export L-histidine and microorganisms having them were selected.

Among them, in consideration of the biosafety level and securing possibility at a level applicable to producing strains among them, as shown in Table 1 below, LysE-based 1 kind, Wex-based 3 kinds, BrnFE-based 2 kinds of proteins, genes encoding them, and microorganisms comprising them were selected:

**[Table 1]**

| L-histidine export candidate list | | | | | | | |
|---|---|---|---|---|---|---|---|
| | No. | Strain | Protein Ref Seq. | gDNA Ref Seq. | Biosafety level | Amino acid sequence | Nucleic acid sequence |
| Wex based | 1 | *Herbaspiri llum aquaticum* (KCTC42 001) | WP_0887 57482.1 | NZ_NJGV 01000035 .1 | 1 | SEQ ID NO: 1 | SEQ ID NO: 2 |
| | 2 | *Cupriavid* us *pinatubon ensis* (KCTC22 125) | WP_0416 80244.1 | CP00009 1.1 | 1 | SEQ ID NO: 3 | SEQ ID NO: 4 |
| | 3 | *Kluyvera cryocresc* ens(KCTC 2580) | WP_0522 83291.1 | NZ_LGHZ 01000014 .1 | 1 | SEQ ID NO: 5 | SEQ ID NO: 6 |
| LysE based | 4 | *Coryneba cterium stationis* (ATCC687 2) | WP_0668 37457.1 | CP01427 9.1 | 1 | SEQ ID NO: 7 | SEQ ID NO: 8 |
| BrnFE based | 5 | *Leucobact er salsicius* (KCTC 19 904) | WP_0261 39602.1 | NZ_AOC N0100002 2.1 | 1 | SEQ ID NO: 9 | SEQ ID NO: 11 |
| | | | WP_0838 79221.1 | | | SEQ ID NO: 10 | |
| | 6 | *Dermabac ter vaginalis* (KCTC39 585) | WP_0652 48528.1 (DvaF) | NZ_CP01 2117.1 | 1 | SEQ ID NO: 12 | SEQ ID NO: 14 |
| | | | WP_0652 48527.1(D vaE) | | | SEQ ID NO: 13 | SEQ ID NO: 15 |
| | | | DvaFE | | | SEQ ID NO: 12 and 13 | SEQ ID NO: 16 (DvaFE operon) |

| | | | | | | | |
|---|---|---|---|---|---|---|---|
| (In Table 1 above, biosafety level is based on the microbial pathogenicity index (level 1-4) defined by U. S. Centers for Disease Control and Prevention (the lower the level, the safer) | | | | | | | |

### Example 2. Production of foreign L-Histidine export gene candidate-introduced vectors and recombinant Corynebacterium sp. strains introducing the same

Six kinds of vectors for introducing the 6 kinds of the foreign L-histidine export gene candidates selected in Example 1 into Corynebacterium sp. strains were produced.

### Example 2-1. Production of vector pDZΔN2131 for inserting target gene

In order to introduce foreign L-histidine export gene candidates, NCgl2131 gene among genes encoding transposon of Corynebacterium glutamicum was used as an insertion site (Journal of Biotechnology 104, 5-25 Jorn Kalinowski et al, 2003). In addition, it was designed to express the foreign L-histidine export gene candidates under a promoter of a Corynebacterium-derived gapA gene (hereafter, PgapA, SEQ ID NO: 17).

In order to substitute the NCgl2131 gene with the export genes, NCgl2131-deleted and target gene-inserted vectors were produced. In order to produce the vectors, PCR was performed using the primer pair of SEQ ID NO: 18 and SEQ ID NO: 19, and SEQ ID NO: 20 and SEQ ID NO: 21, respectively, with chromosome of Corynebacterium glutamicum strain ATCC13032 as a template. As polymerase for PCR reaction, PfuUltraTM high-reliability DNA polymerase (Stratagene) was used, and the PCR conditions were as follows: as the result of repeating denaturation at 95°C, for 30 seconds; annealing at 55°C, for 30 seconds; and polymerization at 72°C, for 2 minutes, 28 times, and then performing polymerization at 72°C for 5 minutes, DNA fragments of del-N2131L (SEQ ID NO: 22) of 531bp and del-N2131R (SEQ ID NO: 23) of 555bp, respectively, were obtained. The obtained DNA products were purified using PCR Purification kit of QIAGEN company, and then were cloned using pDZ vector (Korean Registered Patent No. 10-0924065) and TaKaRa' s Infusion Cloning Kit, and vector pDZΔN2131 for NCgl2131 gene deletion and target gene insertion was produced.

### Example 2-2. Production of 6 kinds of L-histidine export gene candidate-introduced vectors

The base sequence information of the gene (hereinafter, haq, SEQ ID NO: 2) encoding Herbaspirillum aquaticum-derived protein (hereinafter, Haq, SEQ ID NO: 1) was obtained from U. S. National Institutes of Health GenBank (NIH GenBank). In order to amplify haq, PCR was performed using the primer pair of SEQ ID NO: 24 and SEQ ID NO: 25 with chromosomal DNA of the Herbaspirillum aquaticum strain (KCTC42001) as a template. As polymerase for PCR reaction, PfuUltraTM high-reliability DNA polymerase (Stratagene) was used, and the PCR conditions were as follows: as the result of repeating denaturation at 95°C, for 30 seconds; annealing at 55°C, for 30 seconds; and polymerization at 72°C, for 2 minutes, 28 times, and then performing polymerization at 72°C for 5 minutes, haq fragments of 977bp comprising haq (SEQ ID NO: 2) of 945bp were obtained. In order to obtain PgapA fragments connectable to haq, PCR was performed using the primer pair of SEQ ID NO: 26 and SEQ ID NO: 27 with chromosome of ATCC13032 as a template. As polymerase for PCR reaction, PfuUltraTM high-reliability DNA polymerase (Stratagene) was used, and the PCR conditions were as follows: as the result of repeating denaturation at 95°C, for 30 seconds; annealing at 55°C, for 30 seconds; and polymerization at 72°C, for 1 minutes, 28 times, and then performing polymerization at 72°C for 5 minutes, PgapA fragments of 441bp comprising PgapA (SEQ ID NO: 17) of 409bp were obtained. The obtained haq fragments and PgapA fragments, and the pDZΔN2131 vector cleaved with Scal restriction enzyme were cloned using Gibson assembly (DG Gibson et al., NATURE METHODS, VOL.6 NO.5, MAY 2009, NEBuilder HiFi DNA Assembly Master Mix) method, to obtain a recombinant plasmid, and this was named pDZΔN2131-PgapA-Haq.

The base sequence information of the gene (hereinafter, cpi, SEQ ID NO: 4) encoding Cupriavidus pinatubonensis-derived protein (hereinafter, Cpi, SEQ ID NO: 3) was obtained from U. S. National Institutes of Health GenBank (NIH GenBank). In order to amplify Cupriavidus pinatubonensis-derived cpi, PCR was performed using the primer pair of SEQ ID NO: 28 and SEQ ID NO: 29 with chromosomal DNA of the Cupriavidus pinatubonensis strain (KCTC22125) as a template. As polymerase for PCR reaction, PfuUltraTM high-reliability DNA polymerase (Stratagene) was used, and the PCR conditions were as follows: as the result of repeating denaturation at 95°C, for 30 seconds; annealing at 55°C, for 30 seconds; and polymerization at 72°C, for 2 minutes, 28 times, and then performing polymerization at 72°C for 5 minutes, cpi fragments of 977bp comprising cpi (SEQ ID NO: 4) of 945bp were obtained. In order to obtain PgapA fragments connectable to cpi, PCR was performed using the primer pair of SEQ ID NO: 26 and SEQ ID NO: 30 with chromosome of ATCC13032 as a template. As polymerase for PCR reaction, PfuUltraTM high-reliability DNA polymerase (Stratagene) was used, and the PCR conditions were as follows: as the result of repeating denaturation at 95°C, for 30 seconds; annealing at 55°C, for 30 seconds; and polymerization at 72°C, for 1 minutes, 28 times, and then performing polymerization at 72°C for 5 minutes, PgapA fragments of 441bp comprising PgapA (SEQ ID NO: 17) of 409bp were obtained. The obtained cpi fragments and PgapA fragments, and the pDZΔN2131 vector cleaved with Scal restriction enzyme were cloned using Gibson assembly method, to obtain a recombinant plasmid, and this was named pDZΔN2131-PgapA-Cpi.

The base sequence information of the gene (hereinafter, *kcr*, SEQ ID NO: 6) encoding *Kluyvera cryocrescens*-derived protein (hereinafter, Kcr, SEQ ID NO: 5) was obtained from U. S. National Institutes of Health GenBank (NIH GenBank). In order to amplify *Kluyvera cryocrescens*-derived *kcr*, PCR was performed using the primer pair of SEQ ID NO: 31 and SEQ ID NO: 32 with chromosomal DNA of the *Kluyvera cryocrescens* strain (KCTC2580) as a template. As polymerase for PCR reaction, PfuUltraTM high-reliability DNA polymerase (Stratagene) was used, and the PCR conditions were as follows: as the result of repeating denaturation at 95°C, for 30 seconds; annealing at 55°C, for 30 seconds; and polymerization at 72°C, for 2 minutes, 28 times, and then performing polymerization at 72°C for 5 minutes, kcrfragments of 914bp comprising *kcr* (SEQ ID NO: 6) of 882bp were obtained. In order to obtain PgapA fragments connectable to *kcr*, PCR was performed using the primer pair of SEQ ID NO: 26 and SEQ ID NO: 33 with chromosome of ATCC13032 as a template. As polymerase for PCR reaction, PfuUltraTM high-reliability DNA polymerase (Stratagene) was used, and the PCR conditions were as follows: as the result of repeating denaturation at 95°C, for 30 seconds; annealing at 55°C, for 30 seconds; and polymerization at 72°C, for 1 minutes, 28 times, and then performing polymerization at 72°C for 5 minutes, PgapA fragments of 441bp comprising PgapA (SEQ ID NO: 17) of 409bp were obtained. The obtained *kcr* fragments and PgapA fragments, and the pDZΔN2131 vector cleaved with Scal restriction enzyme were cloned using Gibson assembly method, to obtain a recombinant plasmid, and this was named pDZΔN2131-PgapA-Kcr.

The base sequence information of the gene (hereinafter, *cst,* SEQ ID NO: 8) encoding *Corynebacterium stationis*-derived protein (hereinafter, Cst, SEQ ID NO: 7) was obtained from U. S. National Institutes of Health GenBank (NIH GenBank). In order to amplify *Corynebacterium stationis*-derived *cst,* PCR was performed using the primer pair of SEQ ID NO: 34 and SEQ ID NO: 35 with chromosomal DNA of the *Corynebacterium stationis* strain (ATCC6872) as a template. As polymerase for PCR reaction, PfuUltraTM high-reliability DNA polymerase (Stratagene) was used, and the PCR conditions were as follows: as the result of repeating denaturation at 95°C, for 30 seconds; annealing at 55°C, for 30 seconds; and polymerization at 72°C, for 2 minutes, 28 times, and then performing polymerization at 72°C for 5 minutes, *cst* fragments of 749bp comprising *cst* (SEQ ID NO: 8) of 717bp were obtained. In order to obtain PgapA fragments connectable to *cst,* PCR was performed using the primer pair of SEQ ID NO: 26 and SEQ ID NO: 36 with chromosome of ATCC13032 as a template. As polymerase for PCR reaction, PfuUltraTM high-reliability DNA polymerase (Stratagene) was used, and the PCR conditions were as follows: as the result of repeating denaturation at 95°C, for 30 seconds; annealing at 55°C, for 30 seconds; and polymerization at 72°C, for 1 minutes, 28 times, and then performing polymerization at 72°C for 5 minutes, PgapA fragments of 441bp comprising PgapA (SEQ ID NO: 17) of 409bp were obtained. The obtained *cst* fragments and PgapA fragments, and the pDZΔN2131 vector cleaved with Scal restriction enzyme were cloned using Gibson assembly method, to obtain a recombinant plasmid, and this was named pDZΔN2131-PgapA-Cst.

The base sequence information of the operon (hereinafter, *Isa*, SEQ ID NO: 11) encoding *Leucobacter salsicius*-derived protein (hereinafter, LsaFE, SEQ ID NO: 9, 10) was obtained from U. S. National Institutes of Health GenBank (NIH GenBank). In order to amplify *Leucobacter salsicius*-derived *Isa,* PCR was performed using the primer pair of SEQ ID NO: 37 and SEQ ID NO: 38 with chromosomal DNA of the *Leucobacter salsicius* strain (KCTC19904) as a template. As polymerase for PCR reaction, PfuUltraTM high-reliability DNA polymerase (Stratagene) was used, and the PCR conditions were as follows: as the result of repeating denaturation at 95°C, for 30 seconds; annealing at 55°C, for 30 seconds; and polymerization at 72°C, for 2 minutes, 28 times, and then performing polymerization at 72°C for 5 minutes, *Isa* fragments of 1080bp comprising *Isa* (SEQ ID NO: 11) of 1048bp were obtained. In order to obtain PgapA fragments connectable to /sa, PCR was performed using the primer pair of SEQ ID NO: 26 and SEQ ID NO: 39 with chromosome of ATCC13032 as a template. As polymerase for PCR reaction, PfuUltraTM high-reliability DNA polymerase (Stratagene) was used, and the PCR conditions were as follows: as the result of repeating denaturation at 95°C, for 30 seconds; annealing at 55°C, for 30 seconds; and polymerization at 72°C, for 1 minutes, 28 times, and then performing polymerization at 72°C for 5 minutes, PgapA fragments of 441bp comprising PgapA (SEQ ID NO: 17) of 409bp were obtained. The obtained *Isa* fragments and PgapA fragments, and the pDZΔN2131 vector cleaved with Scal restriction enzyme were cloned using Gibson assembly method, to obtain a recombinant plasmid, and this was named pDZΔN2131-PgapA-Lsa.

The base sequence information of the operon (hereinafter, *dva,* SEQ ID NO: 16) encoding *Dermabacter vaginalis-derived* protein (hereinafter, DvaFE, SEQ ID NO: 12, 13) was obtained from U. S. National Institutes of Health GenBank (NIH GenBank). In order to amplify *Dermabacter vaginalis*-derived *dva,* PCR was performed using the primer pair of SEQ ID NO: 40 and SEQ ID NO: 41 with chromosomal DNA of the *Dermabacter vaginalis* strain (KCTC39585) as a template. As polymerase for PCR reaction, PfuUltraTM high-reliability DNA polymerase (Stratagene) was used, and the PCR conditions were as follows: as the result of repeating denaturation at 95°C, for 30 seconds; annealing at 55°C, for 30 seconds; and polymerization at 72°C, for 2 minutes, 28 times, and then performing polymerization at 72°C for 5 minutes, *dva* fragments of 1113bp comprising *dva* (SEQ ID NO: 16) of 1081bp were obtained. In order to obtain PgapA fragments connectable to *dva,* PCR was performed using the primer pair of SEQ ID NO: 26 and SEQ ID NO: 42 with chromosome of ATCC13032 as a template. As polymerase for PCR reaction, PfuUltraTM high-reliability DNA polymerase (Stratagene) was used, and the PCR conditions were as follows: as the result of repeating denaturation at 95°C, for 30 seconds; annealing at 55°C, for 30 seconds; and polymerization at 72°C, for 1 minutes, 28 times, and then performing polymerization at 72°C for 5 minutes, PgapA fragments of 441bp comprising PgapA (SEQ ID NO: 17) of 409bp were obtained. The obtained *dva* fragments and PgapA fragments, and the pDZΔN2131 vector cleaved with Scal restriction enzyme were cloned using Gibson assembly method, to obtain a recombinant plasmid, and this was named pDZΔN2131-PgapA-Dva.

### Example 2-3. Production of recombinant strains of the genus Corynebacterium

In order to confirm the L-histidine export ability of the foreign L-histidine export gene candidates, the produced NCgl2131-deleted vector (pDZΔN2131), and the 6 kinds of the foreign L-histidine export gene candidate-introduced vectors (pDZΔN2131-PgapA-Haq, pDZΔN2131-PgapA-Cpi, pDZΔN2131-PgapA-Kcr, pDZΔN2131-PgapA-Cst, pDZΔN2131-PgapA-Lsa, pDZΔN2131-PgapA-Dva) were introduced into Corynebacterium glutamicum ATCC13032 strain, respectively. More specifically, the vectors were transformed into the ATCC13032 strain by electroporation, respectively, and through a secondary crossing process, 7 kinds of recombinant strains, in which the NCgl2131 gene on chromosome was deleted or the L-histidine export gene candidates were substituted, were produced, and they were named ATCC13032ΔN2131 (N2131 gene deletion), ATCC13032ΔN2131::Haq (substitution of N2131 gene with haq), ATCC13032ΔN2131::Cpi (substitution of N2131 gene with *cpi*), ATCC13032ΔN2131::Kcr (substitution of N2131 gene with *kcr*), ATCC13032ΔN2131::Cst (substitution of N2131 gen with *cst*), ATCC13032ΔN2131::Lsa (substitution of N2131 gene with *Isa*), and ATCC13032ΔN2131::Dva (substitution of N2131 gene with *dva*), respectively.

### Example 3. MIC measurement of foreign L-histidine export gene candidate-introduced Corynebacterium sp. strains

In order to confirm retention of the activity of L-histidine export ability of the 7 kinds of recombinant Corynebacterium glutamicum strains (ATCC13032ΔN2131, ATCC13032ΔN2131::Haq, ATCC13032ΔN2131::Cpi, ATCC13032ΔN2131::Kcr, ATCC13032ΔN2131::Cst, ATCC13032ΔN2131::Lsa, and ATCC13032ΔN2131::Dva) produced in Example 2 above, a minimum inhibitory concentration (MIC) experiment using L-histidine was performed. The 7 kinds of strains were cultured in a minimal liquid medium at 30°C for 24 hours, and then they were diluted with cells of 1 X 10³ and 1 X 10⁴, and spotting culturing was performed in a minimal solid medium in which L-histidine was added. The composition of the used minimal solid medium was as follows:

### Minimal medium (pH 7.2)

Glucose 10 g, KH₂PO₄ 1 g, K₂HPO₄ 2 g, MgSO₄ 7H₂O 0.4 g, urea 2 g, (NH₄)₂SO₄ 5 g, NaCl 0.5 g, nicotinamide 5 µg, calcium-pantothenate 0.1 µg, biotin 0.2 µg, thiamine HCl 3 µg, Trace elements solution* 1ml (based on 1 liter of distilled water), Agar 20 g

### *Trace elements solution

Na₂B₄O₇ 10H₂O 0.09 g, (NH₄)₆Mo₇O₂₇ 4H₂O 0.04 g, ZnSO₄ 7H₂O 0.01 g, CuSO₄ 5H₂O 0.27 g, MnCl₂ 4H₂O 0.01 g, FeCl₃ 6H₂O 1 g, CaCl₂ 0.01 g (based on 1 liter of distilled water)

For the minimum inhibitory concentration experiment, L-histidine of 1 g/L was added to the minimal solid medium, and after 48 hours, the growth of cells was observed, and the result was shown in Table 2 below:

**[Table 2]**

| Growth level of foreign L-histidine export gene candidate-introduced Corynebacterium sp. strains in minimal medium comprising L-histidine | | |
|---|---|---|
| Strain | Minimal medium comprising no L-histidine | Minimal medium comprising 1 g/L L-histidine |
| ATCC13032ΔN2131 | ++++ | + |
| ATCC13032ΔN2131::Haq | ++++ | + |
| ATCC13032ΔN2131::Cpi | ++++ | + |
| ATCC13032ΔN2131::Kcr | ++++ | + |
| ATCC13032ΔN2131::Cst | ++++ | + |
| ATCC13032ΔN2131::Lsa | ++++ | + |
| ATCC13032ΔN2131::Dva | ++ | +++ |

| | | |
|---|---|---|
| (In Table 2, the number of + indicates the relative growth level of strains, and each indicates the following: + : single colonies are not formed, but heavy (a form that does not grow into a single colony, but grows in clumps) is formed; ++ : heavy is formed, and single colonies less than 5 are formed; +++ : heavy is formed, and single colonies less than 50 are formed; ++++ : heavy is formed without being distinguished from single colonies) | | |

As shown in Table 2 above, all strains except for the ATCC13032ΔN2131::Dva strain smoothly grew in a minimal medium comprising no L-histidine. However, in the minimal medium comprising L-histidine of 1 g/L, the growth of the L-histidine export candidate genes-introduced strains was insignificant, and only the ATCC13032ΔN2131::Dva strain in which the *Dermabacter vaginalis-derived* gene was introduced showed growth superior to ATCC13032ΔN2131. This, shows that the introduced *Dermabacter vaginalis-derived* protein could have the L-histidine export ability even in the medium comprising L-histidine above the minimum inhibitory concentration.

From this, the *Dermabacter vaginalis-derived* protein Dva was selected as a protein giving resistance to L-histidine above the minimum inhibitory concentration into the Corynebacterium strain, and having L-histidine-specific export ability.

### Example 4. Production of Dermabacter vaginalis-derived gene (dva)-introduced strain based on Corynebacterium-derived L-histidine producing strain (KCCM 80179) and evaluation of L-histidine production ability

In order to confirm the L-histidine export ability of the *Dermabacter vaginalis-derived* protein Dva, the *Dermabacter vaginalis-derived* gene *dva* was introduced into the L-histidine producing strain KCCM 80179 (Korean Patent Publication No. 10-2019-0065984).

For this, the vectors produced in Example 2, pDZΔN2131, and pDZΔN2131-PgapA-Dva, were transformed into the KCCM80179 strain, respectively, and through a secondary crossing process, 2 kinds of strains in which the NCgl2131 gene on chromosome was deleted, or L-histidine export gene candidate (*dva*) was substituted, and this was named KCCM 80179ΔN2131 (NCgl2131 gene deletion) and KCCM 80179ΔN2131-PgapA-Dva (substitution of NCgl2131 gene with *dva*), respectively.

In order to confirm the L-histidine production ability of the produced KCCM 80179ΔN2131 and KCCM 80179ΔN2131-PgapA-Dva strain, they were cultured by the following method: the KCCM 80179ΔN2131 and KCCM 80179ΔN2131-PgapA-Dva strains were cultured in an activation medium for 16 hours, and then each strain was inoculated in a 250 mℓ corner-baffled flask containing a seed medium of 25 mℓ, and was cultured with shaking at 200 rpm at 30 °C for 20 hours. Then, the seed cultured solution of 1 mℓ was inoculated in a 250 mℓ corner-baffled flask containing a production medium of 25 mℓ, and was cultured with shaking at 200 rpm at 30 °C for 48 hours. The composition of the medium used for the culturing was as follows:

### <Activation medium>

Meat juice 1%(w/v), polypeptone 1%(w/v), sodium chloride 0.5%(w/v), yeast extract 1%(w/v), agar 2%(w/v), pH 7.2

### <Seed medium>

Glucose 5%(w/v), bactopeptone 1%(w/v), sodium chloride 0.25%(w/v), yeast extract 1%(w/v), urea 0.4%(w/v), pH 7.2

### <Production medium>

Glucose 10%(w/v), ammonium sulfate 2%(w/v), potassium phosphate monobasic 0.1%(w/v), magnesium sulfate heptahydrate 0.05%(w/v), CSL (corn steep liquor) 2.0%(w/v), biotin 200 µg/L, calcium carbonate, pH 7.2,

After completing the culturing, the L-histidine production (histidine content in medium) was measured by HPLC, and the result was shown in the following Table 3:

**[Table 3]**

| L-histidine production of strain introduced with the KCCM 80179-derived *Dermabacter vaginalis-derived* gene | | | |
|---|---|---|---|
| | Cell OD₆₀₀ | Used glucose (g/L) | Histidine production (g/L) |
| KCCM 80179 | 51.4 | 100 | 14.1 |
| KCCM 80179ΔN2131 | 51.6 | 100 | 13.9 |
| KCCM 80179ΔN2131-PgapA-Dva | 42.6 | 100 | 17.1 |

As shown in Table 3 above, it was confirmed that while the NCgl2131-deleted strain had the L-histidine production ability at a level equivalent to the parent strain, KCCM 80179 strain, the KCCM 80179ΔN2131-PgapA-Dva strain in which the *Dermabacter vaginalis-*derived gene was introduced had the L-histidine production ability increased by 23% and 21% or more, respectively, compared to the NCgl2131-deleted strain and the parent strain.

Through the results of Examples 3 and 4, it was confirmed that not only the resistance to the L-histidine concentration above the minimum inhibitory concentration was increased, but also the L-histidine production ability was significantly increased, through introduction of the *Dermabacter vaginalis-derived* gene. This result demonstrates that the *Dermabacter vaginalis*-derived protein is an L-histidine export protein capable of specifically exporting L-histidine.

### Example 5. Additional securement of Dermabacter vaginalis-derived L-histidine export-like protein

Sine the L-histidine export ability of the *Dermabacter vaginalis-derived* protein was confirmed in Examples 3 and 4 above, in order to additionally secure a similar protein having high homology to the protein and amino acid sequences, BLAST search was performed using the sequence of DvaF (SEQ ID NO: 12) among DvaFE as a query (See Table 4).

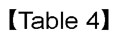

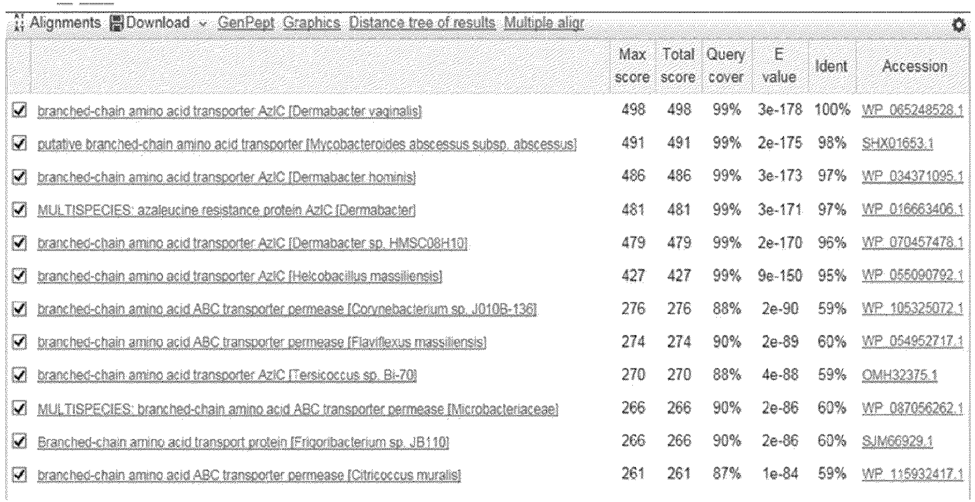

Based on the BLAST search result, 1 kind of the L-histidine export candidate which showed sequence homology of 60% or more and did not belong to the genus *Dermabacter* was additionally selected, and it was shown in the following Table 5:

**[Table 5]**

| L-histidine export additional candidate list | | | | | | |
|---|---|---|---|---|---|---|
| No. | Strain | Protein Ref Seq. | gDNA Ref Seq. | Biosafety level | Protein sequence | Gene sequence |
| 7 | *Helcobacillu* s *massiliensis* | WP_05509 0792.1 (HmaF) | NZ_CYUG0 1000017.1 | 2 | SEQ ID NO: 43 | SEQ ID NO: 45 |
| | | WP_05509 0293.1 (HmaE) HmaFE | | | SEQ ID NO: 44 SEQ ID NO: 43 and 44 | SEQ ID NO: 46 SEQ ID NO: 47 (HmaFE operon) |

### Example 6. Production of additional foreign L-histidine export gene candidate-introduced vector

A vector for introducing the 2 kinds of the L-histidine export gene candidates additionally selected in Example 5 into the strain of the genus Corynebacterium was produced. As same as Example 2, the NCgl2131 gene was used as a defect site, and PgapA was used as a promoter.

The base sequence information of the operon (hereinafter, hma, SEQ ID NO: 47) encoding *Helcobacillus massiliensis-derived* protein (hereinafter, HmaFE, SEQ ID NO: 43, 44) was obtained from U. S. National Institutes of Health GenBank (NIH GenBank). In order to obtain haq DNA, DNA was synthesized using the gene synthesis service of Bionics company. To amplify the synthesized DNA, PCR was performed using the primer pair of SEQ ID NO: 48 and SEQ ID NO: 49. As polymerase for PCR reaction, PfuUltraTM high-reliability DNA polymerase (Stratagene) was used, and the PCR conditions were as follows: as the result of repeating denaturation at 95°C, for 30 seconds; annealing at 55°C, for 30 seconds; and polymerization at 72°C, for 2 minutes, 28 times, and then performing polymerization at 72°C for 5 minutes, hma fragments of 1113bp comprising hma (SEQ ID NO: 47) of 1081bp were obtained. In order to obtain PgapA fragments connectable to hma, PCR was performed using the primer pair of SEQ ID NO: 26 and SEQ ID NO: 50 with chromosome of ATCC13032 as a template. As polymerase for PCR reaction, PfuUltraTM high-reliability DNA polymerase (Stratagene) was used, and the PCR conditions were as follows: as the result of repeating denaturation at 95°C, for 30 seconds; annealing at 55°C, for 30 seconds; and polymerization at 72°C, for 1 minutes, 28 times, and then performing polymerization at 72°C for 5 minutes, PgapA fragments of 441bp comprising PgapA (SEQ ID NO: 17) of 409bp were obtained. The obtained hma fragments and PgapA fragments, and the pDZΔN2131 vector cleaved with Scal restriction enzyme were cloned using Gibson assembly method, to obtain a recombinant plasmid, and this was named pDZΔN2131-PgapA-Hma.

### Example 7. Production of Helcobacillus massiliensis-derived gene (hma)-introduced strain based on L-histidine producing strain (KCCM 80179) and evaluation of L-histidine production ability

In order to confirm the L-histidine export ability of the *Helcobacillus massiliensis-*derived protein Hma, it was introduced into the L-histidine producing strain KCCM 80179 strain.

For this, the vector pDZΔN2131-PgapA-Hma produced in Example 6 was transformed into the KCCM80179 strain by electroporation, and through a secondary crossing process, a strain in which the NCgl2131 gene on chromosome was substituted with the L-histidine export gene candidate was produced, and this was named KCCM 80179ΔN2131-PgapA-Hma (substitution of NCgl2131 gen with hma).

In order to confirm the L-histidine production ability of the produced KCCM 80179ΔN2131-PgapA-Hma strain was cultured by the method performed in Example 4, and the L-histidine production was measured. As a control group, for the KCCM 80179ΔN2131 strain and the KCCM 80179ΔN2131-PgapA-Dva strain produced in Example 4, culturing and measuring the L-histidine production (histidine content in medium) were performed by the same method. The obtained results were shown in Table 6.

**[Table 6]**

| L-histidine production of the strain introduced with the KCCM 80179-derived *Helcobacillus massiliensis-derived* gene | | | |
|---|---|---|---|
| | OD | Used glucose (g/L) | Histidine production (g/L) |
| KCCM 80179 | 50.3 | 100 | 14.0 |
| KCCM 80179ΔN2131 | 51.5 | 100 | 13.9 |
| KCCM 80179ΔN2131-PgapA-Dva | 40.8 | 100 | 17.3 |
| KCCM 80179ΔN2131-PgapA-Hma | 43.1 | 100 | 16.6 |

As shown in Table 6, the KCCM 80179ΔN2131-PgapA-Hma strain had the L-histidine production increased by 19%, 19%, respectively, compared to the NCgl2131-deleted strain (KCCM 80179ΔN2131) and the parent strain, KCCM 80179 strain. This result shows that the *Helcobacillus massiliensis-derived* protein can be also selected as an L-histidine exporter capable of specifically exporting L-histidine.

### Example 8: Production of Hma mutant library using artificial mutation method

In order to obtain mutant Hma with increased L-histidine export ability, a mutant protein expression vector library for primary crossover was produced. For this, hma operons in which base substitution mutation was randomly introduced by Error-prone PCR method using the primer pair of SEQ ID NO: 48 and SEQ ID NO: 49 with the produced pDZ ΔN2131-PgapA-Hma as a template were obtained. For Error-prone PCR, PCR was performed using Genemorphll Random Mutagenesis Kit (Stratagene) under the condition in which 0 to 3.5 mutations per 1kb were introduced into the amplified gene fragments, and the PCR conditions were repeating denaturation at 96°C, for 30 seconds; annealing at 53°C, for 30seconds; and polymerization at 72°C, for 2 minutes, 30 times. In order to obtain PgapA fragments connectable to hma, PCR was performed using the primer pair of SEQ ID NO: 26 and SEQ ID NO: 50 with chromosome of ATCC13032 as a template. As polymerase for PCR reaction, PfuUltraTM high-reliability DNA polymerase (Stratagene) was used, and the PCR conditions were as follows: PgapA fragments were obtained by repeating denaturation at 95°C, for 30 seconds; annealing at 55°C, for 30 seconds; and polymerization at 72°C, for 1 minute, 28 times, and then performing polymerization at 72°C for 5 minutes. The obtained mutant hma operons and PgapA fragments, and the pDZΔN2131 vector cleaved with Scal restriction enzyme were cloned using Gibson assembly (DG Gibson et al., NATURE METHODS, VOL.6 NO.5, MAY 2009, NEBuilder HiFi DNA Assembly Master Mix) method, and transformed into DH5α, and smeared in an LB solid medium comprising kanamycin (25mg/L). As the result of selecting 20 kinds of transformed colonies and then obtaining a plasmid, and analyze the base sequence, it was confirmed that mutations were introduced at positions different from each other at a frequency of 2 mutations/kb on average. The plasmid was extracted by taking about 10,000 transformed E. coli colonies, and this was named pDZΔN2131-PgapA-Hma(mt) library.

### Example 9: Introduction of Hma artificial mutant library and selection of strain having increased L-histidine production ability

The produced pDZΔN2131-PgapA-Hma(mt) library was transformed by homologous chromosome recombination using the produced ATCC13032ΔN2131 strain as a parent strain, and it was smeared in a complex plate medium comprising kanamycin (25mg/L) to secure about 7000 colonies, and each colony was named from ATCC13032ΔN2131-PgapA-Hma(mt)-1 to ATCC13032ΔN2131-PgapA-Hma(mt)-7000.

### <Complex plate medium (pH 7.0)>

Glucose 10g, Peptone 10g, Beef extract 5g, Yeast extract 5g, Brain Heart Infusion 18.5g, NaCl 2.5g, Urea 2g, Sorbitol 91g, Agar 20g (based on 1 liter of distilled water)

For the secured 7000 colonies, the minimum inhibitory concentration (MIC) experiment for L-histidine performed in Example 3 was performed. For efficient screening, it was performed based on a liquid minimal medium comprising L-histidine of 3g. The colonies were inoculated into the liquid minimal medium of 300ul immediately, and were cultured in a 96-deep well plate at 32°C, 1000rpm for 18 hours, and then the OD600 values were measured to select a colony with excellent growth. As a control group, the produced ATCC13032ΔN2131 strain and the ATCC13032ΔN2131::Hma strain were used, and through a primary experiment, 251 were selected, and through a re-repeating experiment, 36 colonies were selected, and the used medium components were as follows.

### Liquid minimal medium (pH 7.2)

Glucose 10g, KH₂PO₄ 1 g, K₂HPO₄ 2 g, MgSO₄ 7H₂O 0.4 g, urea 2 g, (NH₄)₂SO₄ 5 g, NaCl 0.5 g, nicotinamide 5 µg, calcium-pantothenate 0.1 µg, biotin 0.2 µg, thiamine HCl 3 µg, Trace elements solution* 1ml (based on 1 liter of distilled water)

### *Trace elements solution

Na₂B₄O₇ 10H₂O 0.09 g, (NH₄)₆Mo₇O₂₇ 4H₂O 0.04 g, ZnSO₄ 7H₂O 0.01 g, CuSO₄ 5H₂O 0.27 g, MnCl₂ 4H₂O 0.01 g, FeCl₃ 6H₂O 1 g, CaCl₂ 0.01 g (based on 1 liter of distilled water)

For the minimum inhibitory concentration experiment, L-histidine of 3 g/L was added to the medium and culturing was performed for 18hr.

Solid medium-based screening was performed for the selected 36 colonies by the method performed in Example 3, and final screening was conducted for 6 kinds of colonies with increased L-histidine export ability, and the result was shown in Table 7 below:

**[Table 7]**

| Degree of growth of mutation-introduced strains in minimal medium comprising histidine L- | | |
|---|---|---|
| Strain | Minimal medium comprising no L-histidine | Minimal medium comprising 1 g/L L-histidine |
| ATCC13032ΔN2131 | ++++ | + |
| ATCC13032ΔN2131::Hma | ++++ | +++ |
| ATCC13032ΔN2131-PgapA-Hma(mt)-1216 | ++++ | ++++ |
| ATCC13032ΔN2131-PgapA-Hma(mt)-2305 | ++++ | ++++ |
| ATCC13032ΔN2131-PgapA-Hma(mt)-3411 | ++++ | ++++ |
| ATCC13032ΔN2131-PgapA-Hma(mt)-4426 | ++++ | ++++ |
| ATCC13032ΔN2131-PgapA-Hma(mt)-5714 | ++++ | ++++ |
| ATCC13032ΔN2131-PgapA-Hma(mt)-6718 | ++++ | ++++ |

| | | |
|---|---|---|
| (In Table 7, the number of + indicates the relative growth level of strains, and each indicates the following: + : single colonies are not formed, but heavy (a form that does not grow into a single colony, but grows in clumps) is formed; ++ : heavy is formed, and single colonies less than 5 are formed; +++ : heavy is formed, and single colonies less than 50 are formed; ++++ : heavy is formed without being distinguished from single colonies) | | |

As shown in Table 7 above, the NCgl2131-deleted strain (ATCC13032ΔN2131) could not grow smoothly in the minimal medium comprising L-histidine of 1 g/L, but the ATCC13032ΔN2131::Hma strain in which the *Helcobacillus massiliensis-derived* gene was introduced grew smoothly, and by performing solid medium-based screening, the growth level higher than that in the minimal medium comprising histidine of 1 g/L was confirmed for the 6 kinds of strains (ATCC13032ΔN2131-PgapA-Hma(mt)-1216, ATCC13032ΔN2131-PgapA-Hma(mt)-2305, ATCC13032ΔN2131-PgapA-Hma(mt)-3411, ATCC13032ΔN2131-PgapA-Hma(mt)-4426, ATCC13032ΔN2131-PgapA-Hma(mt)-5714, and ATCC13032ΔN2131-PgapA-Hma(mt)-6718 strains).

### Example 10. Production of strains introduced with library selected based on Corynebacterium-derived L-histidine producing strain (KCCM 80179) and evaluation of L-histidine production ability

Vectors for introducing the mutant hma comprised by the 6 kinds of colonies selected in Example 9 into an L-histidine producing strain were produced. For this, PCR was performed using the primer pair of SEQ ID NO: 48 and SEQ ID NO: 49 with chromosomal DNA of ATCC13032ΔN2131-PgapA-Hma(mt)-1216, ATCC13032ΔN2131-PgapA-Hma(mt)-2305, ATCC13032ΔN2131-PgapA-Hma(mt)-3411, ATCC13032ΔN2131-PgapA-Hma(mt)-4426, ATCC13032ΔN2131-PgapA-Hma(mt)-5714, ATCC13032ΔN2131-PgapA-Hma(mt)-6718 as a template to obtain mutant hma. The PCR conditions were repeating denaturation at 96°C, for 30 seconds; annealing at 53°C, for 30 seconds; and polymerization at 72°C, for 2 minutes, 30 times. In order to obtain PgapA fragments connectable to mutant hma, PCR was performed using the primer pair of SEQ ID NO: 26 and SEQ ID NO: 50 with chromosome of ATCC13032 as a template. As polymerase for PCR reaction, PfuUltraTM high-reliability DNA polymerase (Stratagene) was used, and the PCR conditions were as follows: denaturation at 95°C, for 30 seconds; annealing at 55°C, for 30 seconds; and polymerization at 72°C, for 1 minute, were repeated 28 times, and then polymerization at 72°C for 5 minutes was performed to obtain PgapA fragments. The obtained 6 kinds of mutant hma fragments and PgapA fragments, and the pDZΔN2131 vector cleaved with Scal restriction enzyme were cloned using Gibson assembly method to obtain recombinant plasmids, and they were named pDZΔN2131-PgapA-Hma(mt)-1216, pDZΔN2131-PgapA-Hma(mt)-2315, pDZΔN2131- PgapA-Hma(mt)-3411, pDZΔN2131-PgapA-Hma(mt)-4426, pDZΔN2131-PgapA-Hma(mt)-5714, pDZΔN2131-PgapA-Hma(mt)-6718 depending on the derived library colony.

After that, the produced vector was transformed into the KCCM 80179 strain by electroporation, and through a secondary crossing process, 6 kinds of strains in which the 6 kinds of the mutant hma were introduced, respectively, were produced, and they were named KCCM80179ΔN2131-PgapA-Hma(mt)-1216, KCCM80179ΔN2131-PgapA-Hma(mt)-2315, KCCM80179ΔN2131-PgapA-Hma(mt)-3411, KCCM80179ΔN2131-PgapA-Hma(mt)-4426, KCCM80179ΔN2131-PgapA-Hma(mt)-5714, KCCM80179ΔN2131-PgapA-Hma(mt)-6718, respectively.

In order to confirm the L-histidine production ability of the produced 6 kinds of strains, using the KCCM 80179ΔN2131-PgapA-Hma strain produced in Example 7 as a control group, it was measured by the same method as Example 4, and the result was shown in Table 8:

**[Table 8]**

| L-histidine production of 6 kinds of mutation hma-introduced strains derived from KCCM 80179 | | | |
|---|---|---|---|
| | Cell OD₆₀₀ | Used glucose (g/L) | Histidine production (g/L) |
| KCCM 80179 | 51.0 | 100 | 13.9 |
| KCCM 80179ΔN2131 | 51.1 | 100 | 14.0 |
| KCCM 80179ΔN2131-PgapA-Hma | 43.5 | 100 | 16.5 |
| KCCM 80179ΔN2131-PgapA-Hma(mt)-1216 | 50.1 | 100 | 15.1 |
| KCCM 80179ΔN2131-PgapA-Hma(mt)-2315 | 45.8 | 100 | 16.2 |
| KCCM80179ΔN2131 -PgapA-Hma(mt)-3411 | 40.1 | 100 | 16.4 |
| KCCM80179ΔN2131 -PgapA-Hma(mt)-4426 | 44.0 | 100 | 16.2 |
| KCCM80179ΔN2131 -PgapA-Hma(mt)-5714 | 41.5 | 100 | 17.1 |
| KCCM80179ΔN2131 -PgapA-Hma(mt)-6718 | 40.7 | 100 | 18.5 |

As shown in Table 8 above, the NCgl2131-deleted strain (KCCM 80179ΔN2131) had the L-histidine production ability at a level equivalent to the parent strain, KCCM 80179 strain, but the ATCC13032ΔN2131::Hma strain in which the *Helcobacillus massiliensis-derived* gene was introduced had increased L-histidine production ability compared to the NCgl2131-deleted strain and the parent strain, KCCM 80179 strain.

It was confirmed that the 6 kinds of strains in which the 6 kinds of mutant hma were introduced had L-histidine production ability at a level equivalent to or higher than the KCCM 80179ΔN2131-PgapA-Hma strain compared to the NCgl2131-deleted strain and the parent strain, KCCM 80179 strain, and in particular, the KCCM80179ΔN2131-PgapA-Hma(mt)-5714 and KCCM80179ΔN2131-PgapA-Hma(mt)-6718 strains had L-histidine production ability increased by 3.6%, 12.1%, respectively, compared to the wild-type hma-introduced KCCM 80179ΔN2131-PgapA-Hma.

### Example 11. Confirmation of mutant Hma gene mutation with increased L-histidine production ability

In order to confirm the mutation introduced into the Hma of the KCCM80179ΔN2131-PgapA-Hma(mt)-6718 strain of which the effect of increasing L-histidine production ability was confirmed in Example 10, the base sequence of the Hma variant was analyzed. To determine the base sequence, PCR was performed using the primer pair of SEQ ID NO: 18 and SEQ ID NO: 21 with gDNA of the KCCM80179ΔN2131-PgapA-Hma(mt)-6718 strain as a template. Through sequencing, the base sequence of the mutant hma operon and the protein sequence of HmaD or HmaE were confirmed, and they were compared with the amino acid sequence of SEQ ID NO: 43 or SEQ ID NO: 44, and through this, the confirmed mutation information of the amino acid sequence of the mutant HmaFE was shown in Table 9.

**[Table 9]**

| KCCM80179ΔN2131-PgapA-Hma(mt)-6718 strain HmaF, HmaE mutations | | |
|---|---|---|
| Strain | HmaF (SEQ ID NO: 43) | HmaE (SEQ ID NO: 44) |
| KCCM80179ΔN2131-PgapA-Hma(mt)-6718 | 172L, 1124V | Mutation X |

As the result of confirming the sequences, it was confirmed that the mutant HmaFE introduced into the KCCM80179ΔN2131-PgapA-Hma(mt)-6718 strain was a mutant exporter with increased L-histidine export ability as I72L (the 72th isoleucine (Ile, I) of SEQ ID NO: 43 was mutated into leucine (Leu, L)), I124V(the 124th isoleucine (Ile, I) of SEQ ID NO: 43 was mutated into valine (Val, V)) mutations were introduced into HmaD.

Through the result, it was confirmed that not only the resistance to the L-histidine concentration above the minimum inhibitory concentration was increased compared to the wild type, but also the L-histidine producing ability was significantly increased, through introduction of the *Helcobacillus massiliensis-derived* mutant exporter. This result demonstrates that the selected *Helcobacillus massiliensis-derived* mutant protein is a mutant L-histidine export protein capable of specifically exporting L-histidine.

### Example 12. Production of Helcobacillus massiliensis-derived gene-introduced strain based on L-histidine producing strain (CA14-737) and evaluation of L-histidine production ability

In order to confirm the L-histidine export ability of the *Helcobacillus massiliensis-*derived protein Hma mutant, it was introduced into the L-histidine producing strain CA14-737 (KCCM 12411P, Korean Patent Publication No. 10-2019-0065984) strain derived from the wild-type Corynebacterium glutamicum ATCC13032 in which HisG polypeptide mutation in which feedback limitation was resolved by L-histidine was introduced and the L-histidine biosynthesis gene was enhanced.

For this, the 3 kinds of vectors produced in Examples 2 and 10 (pDZΔN2131, pDZΔN2131-PgapA-Hma, pDZΔN2131-PgapA-Hma(mt)-6718) were transformed into the CA14-737 strain, respectively, by electroporation, and through a secondary crossing process, 3 kinds of strains in which the NCgl2131 gene on chromosome was deleted, or the L-histidine export gene was substituted, were produced, and they were named CA14-737ΔN2131, CA14-737ΔN2131-PgapA-Hma, CA14-737ΔN2131-PgapA-Hma(mt)-6718, respectively.

In order to confirm the L-histidine production ability of the produced CA14-737ΔN2131, CA14-737ΔN2131-PgapA-Hma, CA14-737ΔN2131-PgapA-Hma(mt)-6718 strains, they were cultured by the method performed in Example 4, and the L-histidine production (histidine content in medium) was measured, and the result was shown in the following Table 10:

**[Table 10]**

| L-histidine production of the strain introduced with CA14-737-derived *Helcobacillus massiliensis*-derived wild-type gene and variant | | | |
|---|---|---|---|
| | **OD** | **Used glucose (g/L)** | **Histidine production (g/L)** |
| CA14-737 | 90.3 | 100 | 4.1 |
| CA14-737ΔN2131 | 90.5 | 100 | 4.0 |
| CA14-737ΔN2131-PgapA-Hma | 71.1 | 100 | 6.3 |
| CA14-737ΔN2131-PgapA-Hma(mt)-6718 | 61.5 | 100 | 7.3 |

As shown in Table 10, the CA14-737ΔN2131-PgapA-Hma strain in which the *Helcobacillus massiliensis-derived* gene was introduced had the L-histidine production increased by 54% compared with the NCgl2131-deleted strain (CA14-737ΔN2131) and the parent strain, CA14-737 strain, and the CA14-737ΔN2131-PgapA-Hma(mt)-6718 strain in which the mutant hma was introduced had it increased by 78%.

Through this, it was confirmed that all the *Helcobacillus massiliensis-derived* wild-type and mutant proteins could specifically export L-histidine, and the selected mutant protein was an L-histidine export having higher export ability than the wild-type protein once again.

### Example 13. Production of vectors for expressing Helcobacillus massiliensis-derived wild-type gene and variant E. coli

In order to confirm the L-histidine export ability of the *Helcobacillus massiliensis-*derived protein Hma and variant for various strains, vectors capable of expressing the wild-type Hma and Hma variant, respectively, were produced. Each gene was cloned into an E. coli expression vector, pCC1BAC (hereinafter, pBAC, Epicenter corp.), and were designed to express the foreign L-histidine export gene candidates under a promoter of the E. coli strain MG1655-derived yccA gene (hereinafter, PyccA, SEQ ID NO: 51).

In order to secure *Helcobacillus massiliensis-derived* protein Hma and mutant gene fragments, PCR was performed using the primer pair of SEQ ID NO: 52 and SEQ ID NO: 53 with chromosomal DNA of ATCC13032ΔN2131-PgapA-Hma and ATCC13032Δ N2131-PgapA-Hma(mt)-6718 as a template to obtain wild-type and mutant hma DNA fragments. The PCR conditions were repeating denaturation at 96°C, for 30 seconds; annealing at 53°C, for 30 seconds; and polymerization at 72°C, for 2 minutes, 30 times. In order to obtain PyccA fragments, PCR was performed using the primer pair of SEQ ID NO: 54 and SEQ ID NO: 55 with chromosome of MG1655 as a template. As polymerase for PCR reaction, PfuUltraTM high-reliability DNA polymerase (Stratagene) was used, and the PCR conditions were as follows: denaturation at 95°C, for 30 seconds; annealing at 55°C, for 30 seconds; and polymerization at 72°C, for 1 minute were repeated 28 times, and then polymerization at 72°C for 5 minutes was performed to obtain PyccA fragments. The obtained wild-type and mutant hma fragments and PyccA fragments, and the pBAC vector cleaved with EcoRI restriction enzyme were cloned using Gibson assembly method to obtain recombinant plasmids, and they were named pBAC-PyccA-Hma, pBAC-PyccA-Hma(mt)-6718.

### Example 14. Production of Helcobacillus massiliensis-derived Hma wild-type gene and variant-introduced strain based on E. coli-derived L-histidine producing strain and evaluation of L-histidine production ability

In order to confirm the L-histidine export ability of the *Helcobacillus massiliensis-*derived protein Hma variant based on the E. coli-derived L-histidine producing strain, 3 kinds of strains in which the 2 kinds of vectors produced in Example 13 and pBAC vector were introduced into the CA14-9003e strain (MG1655+ hisGr hisL'_Δ ΔpurR) having previously reported genotypes (purR deletion, hisL deletion, hisGr; The directed modification of Escherichia coli MG1655 to obtain histidine-producing mutants; Applied Biochemistry and Microbiology, 2013, Vol. 49, No. 2, pp. 130-135) were produced, and they were named CA14-9003e/pBAC, CA14-9003e/pBAC-PyccA-Hma, CA14-9003e/pBAC-PyccA-Hma(mt)-6718, respectively.

In order to confirm the L-histidine producing ability of the produced CA14-9003e/pBAC, CA14-9003e/pBAC-PyccA-Hma, CA14-9003e/pBAC-PyccA-Hma(mt)-6718 strains, they were cultured by the following method. The strains were cultured in an LB solid medium (comprising chloramphenicol of 25 µg/ml) for 16 hours, and then each strain was inoculated into a 250 mℓ corner-baffled flask containing an LB liquid medium of 25 mℓ, and they were cultured with shaking at 200 rpm, at 37 °C for 20 hours. Then, the seed cultured solution of 1 mℓ was inoculated into a 250 mℓ corner-baffled flask containing an E. coli producing medium (Applied Biochemistry and Microbiology, 2013, Vol. 49, No. 2, pp. 130-135) of 25 mℓ, and they were cultured with shaking at 200 rpm, at 37 °C for 48 hours. The medium used for the culturing was as follows:

### <E. coli production medium>

Glucose 4%(w/v), yeast extract 0.2%(w/v), ammonium sulfate 1.6%(w/v), potassium phosphate dibasic trihydrate 0.06%(w/v), iron sulfate heptahydrate 0.0005%(w/v), magnesium sulfate pentahydrate 0.0005%(w/v), calcium carbonate, pH 7.2,

After completing the culturing, by measuring the L-histidine production (histidine content in the medium) by HPLC, the result was shown in the following Table 11.

**[Table 11]**

| L-histidine production of the strain introduced with CA14-9003e-derived *Helcobacillus massiliensis-derived* wild-type and variant | | | |
|---|---|---|---|
| | OD | Used glucose (g/L) | Histidine production (g/L) |
| CA14-9003e/pBAC | 24.5 | 40 | 2.9 |
| CA14-9003e/pBAC-PyccA-Hma | 17.1 | 40 | 3.7 |
| CA14-9003e/pBAC-PyccA-Hma(mt)-6718 | 14.5 | 40 | 4.8 |

As shown in Table 11, it was confirmed that the mutant Hma-introduced CA14-9003e/pBAC-PyccA-Hma(mt)-6718 strain had L-histidine production ability increased by 30% compared to the wild-type Hma-introduced strain, CA14-9003e/pBAC-PyccA-Hma strain.

Through the result, it was confirmed that the L-histidine export activity out of cells was significantly increased, even when the *Helcobacillus massiliensis-derived* L-histidine export variant into microorganisms other than Corynebacterium sp. strains.

From the above description, those skilled in the art to which the present disclosure pertains will be able to understand that the present invention can be implemented in other specific forms without changing the technical spirit or essential features thereof. In this regard, the examples described above should be understood as illustrative and non-limiting in all respects. The scope of the present disclosure should be construed that all changed or modified forms derived from the meaning and scope of the claims to be described later and equivalent concepts thereof rather than the above detailed description are included in the scope of the present disclosure.

## Claims

1. A mutant L-histidine export protein, in which
(1) the amino acid corresponding to the 72th amino acid residue of the amino acid sequence of SEQ ID NO: 43 is substituted with another amino acid,
(2) the amino acid corresponding to the 124th residue of the amino acid sequence of SEQ ID NO: 43 is substituted with another amino acid, or
(3) both of the (1) and (2) are substituted.

2. The protein according to claim 1,
wherein (1) the amino acid corresponding to the 72th amino acid residue of the amino acid sequence of SEQ ID NO: 43 is substituted with leucine, glycine, proline, alanine, valine or methionine, or
(2) the amino acid corresponding to the 124th residue of the amino acid sequence of SEQ ID NO: 43 is substituted with valine, glycine, proline, alanine, leucine or methionine, or
(3) both of the (1) and (2) are substituted.

3. The protein according to claim 1, wherein the protein has sequence homology of 99% or more to the amino acid sequence of SEQ ID NO: 56.

4. A polynucleotide encoding the protein of any one of claims 1 to 3.

5. A microorganism, comprising the protein of any one of claims 1 to 3 or a polynucleotide encoding the protein.

6. The microorganism according to claim 5, wherein the microorganism has the ability to produce L-histidine.

7. The microorganism according to claim 5, wherein the microorganism is the genus of Corynebacterium or the genus of Escherichia.

8. The microorganism according to claim 7, wherein the microorganism is *Corynebacterium glutamicum* or *Escherichia coli.*

9. A composition for producing L-histidine, comprising
the protein of any one of claims 1 to 3,
a polynucleotide encoding the protein, or
a recombinant microorganism comprising the polynucleotide.

10. A method for producing L-histidine, comprising culturing a microorganism comprising the protein of any one of claims 1 to 3 or a polynucleotide encoding the protein in a medium.

11. The method for producing L-histidine according to claim 10, further comprising recovering L-histidine from the cultured microorganism or medium, after the culturing.
